(19) Europäisches Patentamt
European Patent Office
Office européen des brevets

(11) EP 4 624 561 A1

(12) **EUROPEAN PATENT APPLICATION**
published in accordance with Art. 153(4) EPC

(43) Date of publication:
**01.10.2025 Bulletin 2025/40**

(21) Application number: **23879873.0**

(22) Date of filing: **19.10.2023**

(51) International Patent Classification (IPC):
***C12M 1/26*** *(2006.01)* ***B05C 5/00*** *(2006.01)*
***B05C 11/00*** *(2006.01)* ***B05C 11/10*** *(2006.01)*
***C12M 1/28*** *(2006.01)*

(52) Cooperative Patent Classification (CPC):
**B05C 5/00; B05C 11/00; B05C 11/10; C12M 1/26; C12M 1/28**

(86) International application number:
**PCT/JP2023/037882**

(87) International publication number:
**WO 2024/085230 (25.04.2024 Gazette 2024/17)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **21.10.2022 JP 2022169315**

(71) Applicant: **NIKON CORPORATION**
**Tokyo 140-8601 (JP)**

(72) Inventors:
- **MORIYAMA Masaki**
**Tokyo 108-6290 (JP)**
- **KOBAYASHI Ryo**
**Tokyo 108-6290 (JP)**
- **ISHIZAWA Naoya**
**Tokyo 108-6290 (JP)**
- **TAKUBO Makiko**
**Tokyo 108-6290 (JP)**
- **HAYASHI Seri**
**Tokyo 108-6290 (JP)**
- **TANAKA Shuhei**
**Tokyo 108-6290 (JP)**
- **FUKUTAKE Naoki**
**Tokyo 108-6290 (JP)**
- **NISHIMURA Kumiko**
**Tokyo 108-6290 (JP)**

(74) Representative: **Hoffmann Eitle**
**Patent- und Rechtsanwälte PartmbB**
**Arabellastraße 30**
**81925 München (DE)**

# (54) CHANNEL MEMBER AND FINE OBJECT MANIPULATION DEVICE

(57) Provided is a channel member which is a member including a flow channel which allows fluid to pass through to enable manipulation of a fine object, the channel member including: an inner peripheral portion which is in contact with the fluid; and an outer peripheral portion which holds the inner peripheral portion and has a lower melting temperature than that of the inner peripheral portion. **In** addition, also provided is a fine object manipulation device including: a channel member; a support unit which supports the outer peripheral portion of the channel member; and an illumination unit which irradiates the fine object with light at least partially through the channel member and is arranged above the support unit.

200
220
210
100
116
35
25
230
FLOW CHANNEL MANIPULATION UNIT 255
ILLUMINANCE ADJUSTMENT UNIT 260
SENSITIVITY ADJUSTMENT UNIT 270
LUMINANCE ADJUSTMENT UNIT 280
240
250

FIG.1

EP 4 624 561 A1

## Description

BACKGROUND

1. TECHNICAL FIELD

The present invention relates to a channel member and a fine object manipulation device.

2. RELATED ART

In cell biology research, a specific cell is suctioned from among many cells in a culture vessel. Patent Document 1 discloses a cell suction support system which suctions a target cell by using a chip. Further approaches for cell suction support systems are needed.

Patent Document 1: Japanese Patent Application Publication No. 2016-000007 General Disclosure

Provided is a member including a flow channel which allows fluid to pass through to enable manipulation of a fine object (sometimes referred to as a channel member). The channel member may have an inner peripheral portion which is in contact with the fluid. Any of the channel members may have an outer peripheral portion which holds the inner peripheral portion and has a lower melting temperature than that of the inner peripheral portion.

A material forming the outer peripheral portion may have a lower melting point than that of a material forming any of the inner peripheral portions. The material forming any of the outer peripheral portions may have a lower glass transition point than that of the material forming any of the inner peripheral portions. The material forming any of the outer peripheral portions may have a lower Young's modulus at 25°C than that of the material forming any of the inner peripheral portions. The material forming any of the inner peripheral portions may have a larger light transmittance value at all wavelengths of 400 nm to 700 nm than that of the material forming any of the outer peripheral portions.

The material forming any of the inner peripheral portions may be glass. The material forming any of the outer peripheral portions may be resin.

The fine object may be a phase object. The phase object may be an organism.

At least one of any of the outer peripheral portions or any of the inner peripheral portions may include a light shielding member. The light shielding member may be arranged on an incident surface on which light is incident in any of the outer peripheral portions. Any of the light shielding members may be arranged at a portion where any of the outer peripheral portions and any of the inner peripheral portions are in contact with each other. Any of the light shielding members may be black paint. A ratio of an area of a portion where any of the outer peripheral portions and any of the inner peripheral portions are in contact with each other to a cross-sectional area in a plane orthogonal to a lengthwise direction of any of the inner peripheral portions may be 500 times or less.

Any of the inner peripheral portions may have an elongated tubular shape that allows internal flow of the fluid. Any of the inner peripheral portions may include a distal end region having a certain length including a distal end portion close to the fine object. Any of the inner peripheral portions may include an inner region other than the distal end region. Any of the outer peripheral portions may surround any of the inner peripheral portions in the inner region. **In** any of the outer peripheral portions, a cross-sectional area of a cross section in a plane orthogonal to a lengthwise direction of any of the inner peripheral portions may decrease toward the distal end portion.

Any of the outer peripheral portions may have a portion surrounding any of the inner peripheral portions without being in contact with any of the inner peripheral portions. In any of the channel members, a gap portion serving as a gap may exist between any of the outer peripheral portions and any of the inner peripheral portions. A proportion of an actual volume of any of the outer peripheral portions not including the gap portion to an apparent volume of any of the outer peripheral portions including the gap portion may be 30 to 80%.

Furthermore, provided is a fine object manipulation device. The fine object manipulation device may include any of the channel members. Any of the fine object manipulation devices may include a support unit which supports any of the outer peripheral portions of any of the channel members. Any of the fine object manipulation devices may include an illumination unit which irradiates the fine object with light at least partially through any of the channel members and is arranged above the support unit.

The illumination unit may include a main illumination which irradiates any of the channel members with light along a lengthwise direction of any of the inner peripheral portions. Light incident on any of the inner peripheral portions from the main illumination may be emitted from a distal end portion of any of the inner peripheral portions to illuminate the fine object from directly above. Any of the illumination units may include an auxiliary illumination which radiates ring-shaped light circumferentially surrounding any of the channel members and irradiates the fine object from an oblique direction.

Any of the illumination units may include a light source. Any of the illumination units may include a filter which divides light from the light source into center light and peripheral light surrounding a vicinity of the center light. Any of the illumination units may include a condenser lens which condenses the center light from the filter to cause the center light to be incident on any of the inner peripheral portions and emits light from a distal end portion of any of the inner peripheral portions to illuminate the fine object from directly above, and condenses the peripheral light to illuminate the fine object from an oblique direction.

Any of the illumination units may radiate coherent light. The coherent light may be illumination light having an illumination NA (numerical aperture) of substantially zero. In the coherent light, a width of a coherence function may be sufficiently wider than a wavelength. The light source may be light from a halogen lamp, light from a mercury lamp, light from an LED, or laser light.

Any of the fine object manipulation devices may further include a placement unit on which the fine object is placed. Any of the fine object manipulation devices may further include an imaging unit which is arranged on a surface opposite to any of the channel members with respect to the placement unit and captures a magnified image of the fine object. The imaging unit may image a manipulation performed by the fluid on the fine object close to a distal end portion of any of the inner peripheral portions. The manipulation may be performed by a bubble formed and maintained at any of the distal end portions of any of the inner peripheral portions.

Any of the fine object manipulation devices may further include an illuminance adjustment unit which adjusts illuminance of any of the illumination units. The fine object manipulation device may further include an adjustment unit which adjusts sensitivity of an imaging element or luminance of an image located directly below any of the channel members among imaging elements of any of the imaging units, based on sensitivity of an imaging element or luminance of the image located in a vicinity directly below any of the channel members. The adjustment unit may include a sensitivity adjustment unit and/or a luminance adjustment unit.

Any of the fine object manipulation devices may further include a sensitivity adjustment unit which adjusts sensitivity of an imaging element located directly below any of the channel members among imaging elements of any of the imaging units, based on sensitivity of an imaging element located in a vicinity directly below any of the channel members (for example, to be higher or lower than the sensitivity of the imaging element located in the vicinity directly below the channel member).

Any of the fine object manipulation devices may further include a luminance adjustment unit which adjusts luminance of an image located directly below any of the channel members in the image captured by any of the imaging units, based on luminance of the image located in a vicinity directly below any of the channel members (for example, to be higher or lower than the luminance of the image located in the vicinity directly below the channel member).

The summary clause does not necessarily describe all necessary features of the embodiments of the present invention. The present invention may also be a sub-combination of the features described above.

BRIEF DESCRIPTION OF THE DRAWINGS

Fig. 1 illustrates an example of a configuration of a fine object manipulation device according to the present embodiment.
Fig. 2 illustrates an example of a channel member according to the present embodiment.
Fig. 3A illustrates an example of a method for collecting a fine object according to the present embodiment.
Fig. 3B illustrates an example of the method for collecting the fine object according to the present embodiment.
Fig. 3C illustrates an example of the method for collecting the fine object according to the present embodiment.
Fig. 4A illustrates an example of the channel member according to the present embodiment.
Fig. 4B illustrates an example of the channel member according to the present embodiment.
Fig. 4C illustrates an example of the channel member according to the present embodiment.
Fig. 5A is a diagram for explaining a porosity and a filling rate according to the present embodiment.
Fig. 5B is a diagram for explaining the porosity and the filling rate according to the present embodiment.
Fig. 5C is a diagram for explaining the porosity and the filling rate according to the present embodiment.
Fig. 5D is a diagram for explaining the porosity and the filling rate according to the present embodiment.
Fig. 6A illustrates an example of the channel member according to the present embodiment.
Fig. 6B illustrates an example of the channel member according to the present embodiment.
Fig. 6C illustrates an example of the channel member according to the present embodiment.
Fig. 7A is a diagram for explaining a taper angle of an outer peripheral portion according to the present embodiment.
Fig. 7B illustrates an example of the channel member according to the present embodiment.
Fig. 8 illustrates an example of a configuration of an illumination unit of the fine object manipulation device according to the present embodiment.
Fig. 9 illustrates a perspective view of the fine object manipulation device of Fig. 8.
Fig. 10 illustrates an example of a bright-field image of the fine object according to the present embodiment.
Fig. 11 illustrates another example of the configuration of the illumination unit of the fine object manipulation device according to the present embodiment.

Fig. 12 illustrates a perspective view of the fine object manipulation device of Fig. 11.

Fig. 13A is an example of an image of the fine object captured by the fine object manipulation device according to the present embodiment.

Fig. 13B is a diagram for explaining adjustment of the image captured in Fig. 13A.

DESCRIPTION OF EXEMPLARY EMBODIMENTS

Hereinafter, the present invention will be described through embodiments of the invention, but the following embodiments do not limit the invention according to claims. In addition, not all combination of the features described in the embodiments are necessary for the solution of the invention. Note that in the drawings, the same or similar parts are assigned with same reference signs, and duplicated descriptions may be omitted.

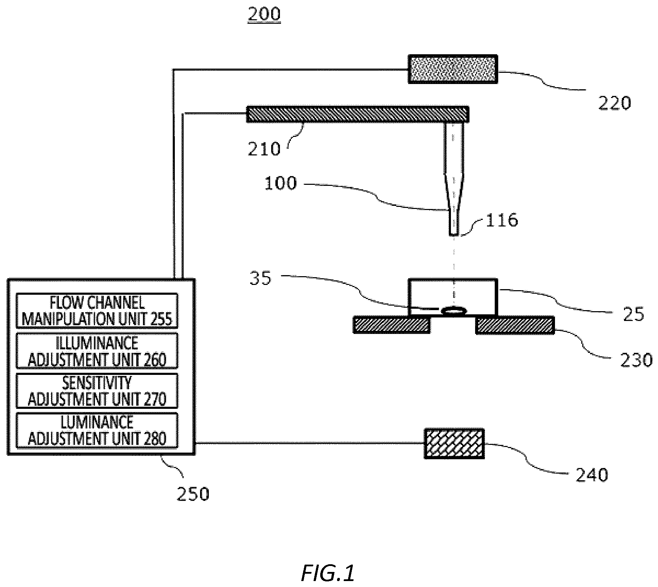

Fig. 1 illustrates an example of a configuration of a fine object manipulation device 200 according to the present embodiment. The fine object manipulation device 200 can manipulate a fine object 35 through a channel member 100. The fine object manipulation device 200 may include all or at least a part of the channel member 100, a support unit 210, an illumination unit 220, a placement unit 230, an imaging unit 240, or a control unit 250.

The channel member 100 is a member including a flow channel. The channel member 100 manipulates a fine object existing in liquid. The channel member 100 may have an inner peripheral portion and an outer peripheral portion. Details will be described later.

The support unit 210 supports the channel member 100. The support unit 210 may support the outer peripheral portion of the channel member 100. For example, the support unit 210 supports the channel member 100. As an example, the support unit 210 supports the channel member 100 through a joint.

The support unit 210 may be connected to a channel member actuator (not illustrated). For example, when the support unit 210 is connected to the channel member actuator, the channel member 100 can operate in any direction of a longitudinal direction, a lateral direction, or a vertical direction. An operation of the channel member actuator may be controlled by the control unit 250 (for example, a flow channel manipulation unit 255 in the control unit 250, which will be described later) or may be controlled manually by an observer.

The support unit 210 may be formed of a material that transmits light. Since the support unit 210 is formed of the material that transmits light, it is possible to prevent the support unit 210 from obstructing illumination when the illumination unit 220 irradiates the fine object 35 and/or the channel member 100. For example, the support unit 210 may be a transparent plastic such as a polycarbonate resin or an acrylate resin, or glass, but is not limited thereto.

The illumination unit 220 irradiates the channel member 100 and/or the fine object 35 with light. The illumination unit 220 may be arranged above the support unit 210. The illumination unit 220 may irradiate the fine object 35 with light at least partially through the channel member 100. For example, the illumination unit 220 irradiates the fine object 35 with light through the flow channel of the channel member 100. For example, the illumination unit 220 has a ring illumination. The illumination unit 220 may radiate coherent light, and details thereof will be described later.

The placement unit 230 places the fine object 35 and/or a container 25 which contains the fine object 35. For example, the placement unit 230 can operate in any direction of the longitudinal direction, the lateral direction, or the vertical direction. A plurality of containers and/or tubes may be mounted on the placement unit 230, but the placement unit 230 is not limited thereto. An operation of the placement unit 230 may be controlled by an information processing apparatus (for example, the control unit 250) such as a computer connected to the placement unit 230, or may be controlled manually by the observer.

The imaging unit 240 images the fine object 35 and generates an image. For example, the imaging unit 240 generates a transmission image of the fine object 35 and generates an image. As an example, the imaging unit 240 images a manipulation which is performed by fluid on the fine object 35 close to a distal end portion of an inner peripheral portion described later. The imaging unit 240 may be a camera (such as a cooling camera) which captures a transmission image.

The imaging unit 240 may be arranged on a surface opposite to the channel member 100 with respect to the placement unit 230. For example, the imaging unit 240 is arranged below the placement unit 230. Since the imaging unit 240 is arranged on the surface opposite to the channel member 100 with respect to the placement unit 230, it is possible to clearly image a state in which the channel member 100 manipulates the fine object 35. The imaging unit 240 may image the fine object 35 by magnifying it at an arbitrary magnification. For example, the imaging unit 240 is connected to a microscope. As an example, the imaging unit 240 captures a magnified image of a manipulation performed by a bubble on the fine object 35 by using an objective lens arranged in an optical path of the microscope. The microscope to be connected may be an inverted microscope apparatus or an upright microscope apparatus.

The imaging unit 240 may be arranged on a surface on a same side as the channel member 100 with respect to the placement unit 230. For example, the imaging unit 240 is arranged exactly above or exactly beside the channel member 100. Even when the imaging unit 240 is arranged exactly above or exactly beside the channel member 100, it is possible to clearly image a state in which the channel member 100 manipulates the fine object 35.

Data of the image generated by the imaging unit 240 may be stored in an information transmission medium such as a hard disk inside the information processing apparatus (for example, the control unit 250) connected to the imaging unit 240 or a CD, or may be output through a printer, a display, or the like further connected to the information processing apparatus.

The control unit 250 performs overall control of an operation of the fine object manipulation device 200. For example, the control unit 250 controls all or at least a part of an operation of the channel member 100, illuminance of the illumination unit 220, the operation of the placement unit 230, sensitivity of an imaging element of the imaging unit 240, or luminance of the image captured by the imaging unit 240. The control unit 250 may be connected to all or a part of the support unit 210, the illumination unit 220, the placement unit 230, or the imaging unit 240. For example, the control unit 250 may be implemented by an information processing apparatus (computer) and/or software. The control unit 250 may include all or at least a part of the flow channel manipulation unit 255, an illuminance adjustment unit 260, a sensitivity adjustment unit 270, or a luminance adjustment unit 280.

The flow channel manipulation unit 255 controls the operation of the channel member actuator to operate the channel member 100. The flow channel manipulation unit 255 may be inside the control unit 250. The flow channel manipulation unit 255 may automatically control the operation of the channel member actuator, or may control the operation in response to an input from the observer.

The illuminance adjustment unit 260 adjusts the illuminance of the illumination unit 220. For example, the illuminance adjustment unit 260 adjusts the illuminance of the illumination unit 220 so as to appropriately illuminate the fine object 35. The illuminance adjustment unit 260 may be inside the control unit 250. When the illumination unit 220 includes a plurality of illuminations and/or regions, the illuminance adjustment unit 260 may adjust illuminance for each of the illuminations and/or regions. The illuminance adjustment unit 260 may adjust the illuminance automatically or may have the observer adjust the illuminance manually.

The sensitivity adjustment unit 270 adjusts the sensitivity of the imaging element of the imaging unit 240. For example, the sensitivity adjustment unit 270 may adjust light receiving sensitivity of the imaging element, and details thereof will be described later. The sensitivity adjustment unit 270 may be inside the control unit 250. The sensitivity adjustment unit 270 may adjust the sensitivity automatically or may have the observer adjust the sensitivity manually. The imaging element may be a complementary metal oxide semiconductor (CMOS) image sensor or a charge coupled device (CCD) image sensor.

The luminance adjustment unit 280 adjusts a luminance value of the image captured by the imaging unit 240. For example, the luminance adjustment unit 280 may amplify only a specific region in the image so as to increase the luminance or may attenuate only the specific region so as to decrease the luminance. The luminance adjustment unit 280 may be inside the control unit 250. The luminance adjustment unit 280 may adjust the luminance automatically or may have the observer adjust the luminance manually. The luminance adjustment unit 280 may adjust brightness or luminosity instead of/in addition to the luminance.

The sensitivity adjustment unit 270 and the luminance adjustment unit 280 may be collectively configured as an adjustment unit. In this case, the adjustment unit may adjust the sensitivity of the imaging element and/or the luminance of the image located directly below the channel member 100 among the imaging elements of the imaging unit 240, based on the sensitivity of the imaging element and/or the luminance of the image located in a vicinity directly below the channel member 100.

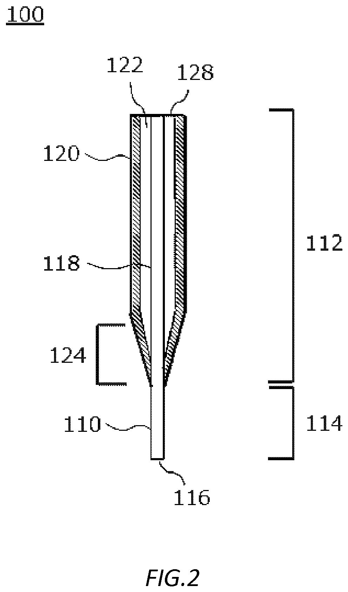

Fig. 2 illustrates an example of the channel member 100 according to the present embodiment. The channel member 100 can manipulate the fine object 35 existing in the liquid by allowing fluid to pass through the flow channel. The fluid that is suctioned (aspirated) or to be discharged (supplied) passes through the flow channel. The flow channel is provided so as to pass through the channel member 100 in a lengthwise direction.

The fluid may be gas and/or liquid. The gas may be air and may contain moisture. The gas may be sterile. For example, gas having passed through the channel member 100 is discharged into liquid to form a bubble, and the fine object 35 existing in the liquid can be manipulated by using the bubble. The flow channel may allow the fine object 35 to pass through together with the fluid.

The fine object 35 may be a phase object. The phase object is an object which, when light is incident, gives a change in phase to the light. The phase object may be a transparent object. For example, the phase object is an organism, a droplet structure (micelles, emulsions, or the like), or a microscopic floating object (such as dust). The organism may be an organic organism. For example, the organism is a cell. As an example, the cell is an animal cell or a plant cell. As an example, the cell is a living cell or a dead cell.

The organism may be a minute organism other than a cell. As an example, the minute organism may be a microorganism, fungus, algae, virus, biological tissue, or spheroid. The minute organism may include an intracellular organelle.

The channel member 100 may have an inner peripheral portion 110 and an outer peripheral portion 120.

The inner peripheral portion 110 has a cavity serving as a fluid flow channel and is in contact with the fluid. The flow channel may have an elongated tubular or cylindrical shape that allows internal flow of the fluid aspirated or to be supplied. For example, the inner peripheral portion 110 has a straw shape or a capillary shape.

A length of the inner peripheral portion 110 in the lengthwise direction may be 1 mm or more and 50 mm or less. The length of the inner peripheral portion 110 in the lengthwise direction may be 5 mm or more and 30 mm or less. The length of the inner peripheral portion 110 in the lengthwise direction may be 10 mm or more and 20 mm or less.

An inner diameter of the flow channel may be 1 μm or more and 10,000 μm or less. The inner diameter of the flow channel may be 10 μm or more and 1,000 μm or less. The inner diameter of the flow channel may be 20 μm or more and 500 μm or less. When the inner diameter and/or the length in the lengthwise direction of the flow channel is within the above range, the channel member 100 can hold a certain amount or more of fluid, making it easier to manipulate the fine object 35. In addition, the channel member 100 is not so long that a manipulation of guiding a distal end portion 116 to an intended position becomes difficult.

The inner peripheral portion 110 may include an inner region 112 and a distal end region 114. The inner region 112 is a region surrounded by the outer peripheral portion 120. The distal end region 114 is a region not surrounded by the outer peripheral portion 120 and has a certain length. The distal end region 114 includes the distal end portion 116 close to the fine object 35. The distal end portion 116 may be arranged in the liquid in which the fine object 35 is immersed. The flow channel may introduce gas into the distal end portion 116, so that the distal end portion 116 forms a bubble. Details of a method of manipulating the fine object 35 by the distal end portion 116 forming a bubble will be described later. Here, the term "close" may mean a distance that allows physical influence on the fine object 35. As an example, the term "close" is a distance at which the fine object 35 and the distal end portion 116 can be brought into contact with each other through the bubble formed at the distal end portion 116.

A distal end surface of the distal end portion 116 may be perpendicular to the lengthwise direction of the inner peripheral portion 110. Alternatively, the distal end surface may be an inclined surface relative to a plane perpendicular to the lengthwise direction. An angle formed by the plane perpendicular to the lengthwise direction and the inclined surface may be 20 degrees or less.

The inner peripheral portion 110 may be a glass tube. For example, the glass tube is molded by a known glass molding technique such as a hand drawing method, a Danner method, or a Vello method. The inner peripheral portion 110 may be a resin tube, but is desirably higher in strength than the outer peripheral portion 120.

The outer peripheral portion 120 holds the inner peripheral portion 110 by surrounding an entire or a part of the inner peripheral portion 110 from an outside, thereby protecting the inner peripheral portion 110. In Fig. 2, the outer peripheral portion 120 is a region indicated by oblique hatching. The outer peripheral portion 120 is supported by the support unit 210 at an end portion 128 opposite to the distal end portion 116. Accordingly, the channel member 100 is supported. A length of the outer peripheral portion 120 in the lengthwise direction may be 2 mm or more and 100 mm or less. The length of the outer peripheral portion 120 in the lengthwise direction may be 10 mm or more and 60 mm or less. The length of the outer peripheral portion 120 in the lengthwise direction may be 20 mm or more and 40 mm or less.

When the length of the outer peripheral portion 120 in the lengthwise direction is within the above range, the outer peripheral portion 120 can appropriately hold and protect the inner peripheral portion 110. When the outer peripheral portion 120 is excessively short, it is difficult to hold and protect the inner peripheral portion 110. When the outer peripheral portion 120 is excessively long, it is difficult to perform a manipulation of guiding the distal end portion 116 to an intended position.

The outer peripheral portion 120 may surround the inner peripheral portion 110 in the inner region 112. For example, the outer peripheral portion 120 surrounds the inner peripheral portion 110 with a surrounding portion 124 of the inner region 112 on the distal end region 114 side. The outer peripheral portion 120 may not surround the inner peripheral portion 110 of the inner region 112 on the side opposite to the distal end region 114 side. The inner peripheral portion 110 may have a protruding portion 118 which protrudes from the outer peripheral portion 120 toward the opposite side. The protruding portion 118 is a portion of the inner peripheral portion 110 surrounded by the outer peripheral portion 120 and a gap portion 122 to be described later.

The outer peripheral portion 120 may surround the inner peripheral portion 110 in contact with a part or a whole of the inner peripheral portion 110. For example, the outer peripheral portion 120 has a portion surrounding the inner peripheral portion 110 without being in contact with a surface other than a contact surface with the inner peripheral portion 110. In this case, the gap portion 122 serving as a gap exists between the inner peripheral portion 110 and the outer peripheral portion 120. In this case, the outer peripheral portion 120 is a hollow member.

The outer peripheral portion 120 may have a cylindrical or polygonal columnar shape in which a central portion corresponding to the inner peripheral portion 110 and the gap portion 122 is a gap. In this case, in the outer peripheral portion 120, at least a part of a cylinder may be a cone or a truncated cone, and at least a part of a polygonal column may be a polygonal pyramid or a polygonal truncated cone. Here, central axes of the cone, the truncated cone, the polygonal pyramid, and the polygonal truncated cone may coincide with a central axis of the inner peripheral portion 110 in the lengthwise direction.

For example, in a portion of the inner region 112 on the distal end region 114 side (for example, all or a part of the surrounding portion 124), the outer peripheral portion 120 has a tapered shape in which an area of a cross section in a plane orthogonal to the lengthwise direction of the inner peripheral portion 110 decreases toward the distal end portion 116. Since the outer peripheral portion 120 has a tapered shape in the portion of the inner region 112 on the distal end region 114 side, when the illumination unit 220 irradiates the fine object 35, it is possible to brightly illuminate the fine object 35 present in the vicinity directly below the channel member 100 (vertically below the channel member 100 in the lengthwise direction). Details thereof will be described later.

In Fig. 2, an end surface (an upper end surface in Fig. 2) of the outer peripheral portion 120 on a side opposite to the distal end portion 116 coincides with an end surface (an upper end surface in Fig. 2) of the inner peripheral portion 110 on the opposite side (the end portion 128 in Fig. 2), but positions of the end surface of the outer peripheral portion 120 and the end surface of the inner peripheral portion 110 are not limited thereto. For example, the upper end surface of the inner peripheral portion 110 may be located below the upper end surface of the outer peripheral portion 120. In this case, the outer peripheral portion 120 is in a form of a container capable of holding fluid in the gap portion 122. Here, after the fluid and/or the fine object 35 is collected, the distal end region 114 of the channel member 100 is folded or cut, and sealed as necessary, whereby the outer peripheral portion 120 may be used as it is as a container for storing the fine object 35 or the liquid.

The outer peripheral portion 120 may be formed of a material that is easier to mold than that of the inner peripheral portion 110. The outer peripheral portion 120 may use, as a material, resin that is easier to mold than glass. The molding of the outer peripheral portion 120 is insert molding or injection molding. In a case of insert molding, first, the inner peripheral portion 110 (for example, a glass tube) is set in a mold. Next, the mold is filled with the material forming the outer peripheral portion 120.

After the outer peripheral portion 120 is molded, the channel member 100 may be manufactured by causing the inner peripheral portion 110 to penetrate the distal end of the outer peripheral portion 120. In this case, since the channel member 100 can be manufactured after the inner peripheral portion 110 and the outer peripheral portion 120 are separately prepared, the channel member 100 as a member desired by the observer can be manufactured.

The material forming the outer peripheral portion 120 may have a melting temperature lower than that of the material forming the inner peripheral portion 110. For example, the material forming the outer peripheral portion 120 has a melting point lower than that of the material forming the inner peripheral portion 110. As a result, the material forming the outer peripheral portion 120 can be melted at a lower temperature than the material forming the inner peripheral portion 110, and molding into an arbitrary shape is facilitated.

For example, the material forming the outer peripheral portion 120 has a glass transition point lower than that of the material forming the inner peripheral portion 110. As a result, the material forming the outer peripheral portion 120 is softer at a lower temperature than the material forming the inner peripheral portion 110, and thus molding into an arbitrary shape is facilitated. Measurement of the glass transition point may be performed by using a differential scanning calorimeter, but is not limited thereto.

The material forming the outer peripheral portion 120 may be more easily deformed than the material forming the inner peripheral portion 110. For example, the material forming the outer peripheral portion 120 has a lower Young's modulus at room temperature (for example, about 20°C, about 25°C, or about 30°C, or the like) than that of the material forming the inner peripheral portion 110. Young's modulus is a value indicating a relationship between strain and stress, and the smaller the value, the more easily a material is deformed. As a result, the material forming the outer peripheral portion 120 is more easily deformed than the material forming the inner peripheral portion 110, and molding into an arbitrary shape is facilitated. Measurement of Young's modulus may be performed according to ASTM D638 standards, but is not limited thereto.

The material forming the inner peripheral portion 110 may have a larger light transmittance value at a wavelength of 400 nm, 450 nm, 500 nm, 550 nm, 600 nm, 650 nm, or 700 nm than that of the material forming the outer peripheral portion 120. The material forming the inner peripheral portion 110 may have a larger light transmittance value at all wavelengths in a visible light region of wavelengths of 400 nm to 700 nm than that of the material forming the outer peripheral portion 120. The material forming the inner peripheral portion 110 may have a smaller light scattering property at a wavelength of 400 nm, 450 nm, 500 nm, 550 nm, 600 nm, 650 nm, or 700 nm than that of the material forming the outer peripheral portion 120. Measurement of the light transmittance and/or the light scattering property may be performed by using a spectrophotometer, but is not limited thereto.

Since the material forming the inner peripheral portion 110 has a larger light transmittance value than that of the material forming the outer peripheral portion 120, the material forming the inner peripheral portion 110 easily transmits light, which is advantageous for the illumination unit 220 to irradiate the channel member 100 with light and illuminate the fine object 35 through the inner peripheral portion 110.

The material forming the inner peripheral portion 110 may have a smaller refractive index value at room temperature than that of the material forming the outer peripheral portion 120. A difference in refractive index between the material forming the inner peripheral portion 110 and the material forming the outer peripheral portion 120 may be preferably 0.2 or more. The difference in refractive index may be 0.3 or more. The difference in refractive index may be 0.5 or more. Measurement of the refractive index of light may be performed by using a critical angle method, but is not limited thereto.

Since the refractive index of the material forming the outer peripheral portion 120 is larger by 0.2 or more than the refractive index of the material forming the inner peripheral portion 110, even when light is about to be incident on the inner peripheral portion 110 from the outer peripheral portion 120, the light can be totally reflected at a boundary between the outer peripheral portion 120 and the inner peripheral portion 110. Therefore, it is possible to prevent light from entering the inner peripheral portion 110 from the outer peripheral portion 120.

The material forming the inner peripheral portion 110 can be used without limitation as long as the material is a material that can be precisely processed and has high strength. For example, the material forming the inner peripheral portion 110 is glass or metal. As an example, the glass is borosilicate glass, soda lime glass, or the like. As an example, the metal is stainless steel, a titanium alloy, an aluminum alloy, or the like. The borosilicate glass has a melting point of about 820°C, and the aluminum has a melting point of about 660°C.

The material forming the outer peripheral portion 120 can be used without limitation as long as the material is a material that can be molded. For example, the material forming the outer peripheral portion 120 is resin. As an example, the resin may be a polypropylene resin, a polyethylene resin, a polystyrene resin, a PET resin, a polyvinyl chloride resin, a polycarbonate resin, or the like, but is not limited thereto. The melting point of the polycarbonate resin is about 150°C.

For example, when an outer shape of the channel member 100 is reproduced only with glass, it is difficult to mass-produce a same shape, and it is also difficult to shorten a length in the lengthwise direction. Furthermore, it is difficult to reproduce, with glass, an arbitrary shape that can be reproduced with resin. In addition, for example, when the outer shape of the channel member 100 is reproduced only with resin, an optical characteristic and a strength in a fine shape are insufficient as compared with those of glass.

In this regard, the channel member 100 includes the outer peripheral portion 120 having low strength but easy molding and the inner peripheral portion 110 having high strength but difficult molding, so that it is possible to utilize advantages of both the inner peripheral portion 110 and the outer peripheral portion 120. The channel member 100 obtained in this manner can easily manipulate the fine object 35, and can be manufactured inexpensively in large quantities.

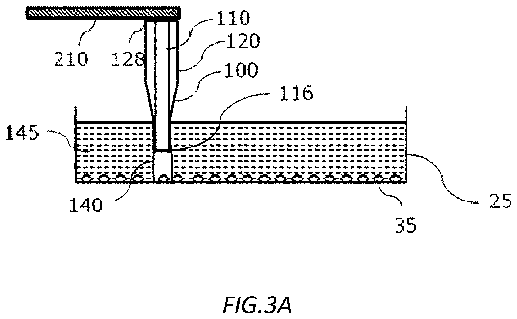

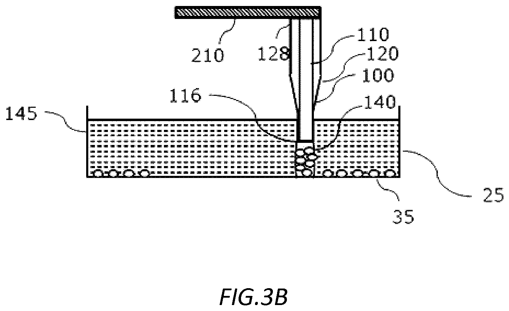

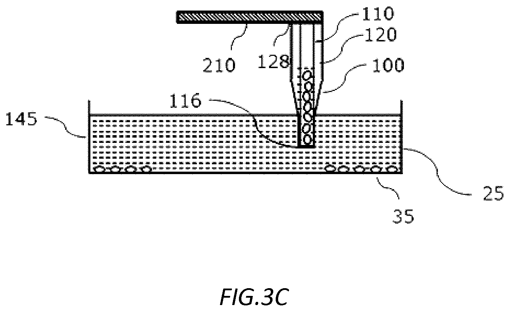

Figs. 3A, 3B, and 3C illustrate an example of a method for collecting the fine object 35 according to the present embodiment. In Figs. 3A, 3B, and 3C, a case of adherent cells is illustrated as an example of the fine object 35. In Fig. 3A, the adherent cells are cultured in a solid phase present on an inner bottom surface of the transparent container 25. The adherent cells may be immersed in liquid 145 in the container 25. The liquid 145 may be a complete medium, a basal medium, or a buffer solution. When the fine object 35 is not an organism or is a dead cell, the present invention is not limited thereto, and water, an organic solvent, or the like may be used.

In Fig. 3A, a pump (not illustrated) connected to the end portion 128 of the inner peripheral portion 110 on the side opposite to the distal end portion 116 supplies gas to the flow channel of the inner peripheral portion 110. Accordingly, a bubble 140 are formed at the distal end portion 116. The formed the bubble 140 contact the adherent cells. The pump regulates supply and aspiration of the gas to maintain the bubble 140 formed at the distal end portion 116. The adherent cells may be manipulated by the bubble 140 formed and maintained at the distal end portion 116. By maintaining the bubble 140, the manipulation of the adherent cells is easily performed.

Next, in Fig. 3B, the support unit 210 connected to the end portion 128 of the inner peripheral portion 110 on the side opposite to the distal end portion 116 moves the channel member 100 along a surface of the solid phase while bringing the bubble 140 into contact with the adherent cells. Fig. 2 illustrates a case where the support unit 210 moves the channel member 100 from left to right, but a direction in which the support unit 210 moves the channel member 100 is not limited as long as the direction is a direction parallel to the surface of the solid phase.

When the support unit 210 moves the channel member 100, the bubble 140 move and the adherent cells adhere to the bubble 140, and/or an external force is applied, so that the adherent cells can be detached from the solid phase. At this time, the detached adherent cells adhere to a gas-liquid interface of the bubble 140. By controlling the pump, it is possible to control a pressure and/or volume of the supplied and aspirated gas. Accordingly, since a size of the bubble 140 can be changed, the adherent cells within a desired range can be detached. Note that instead of the support unit 210 moving the channel member 100 along the surface of the solid phase, the placement unit 230 may be moved to detach the adherent cells from the solid phase.

Next, in Fig. 3C, the pump aspirates gas in the flow channel of the inner peripheral portion 110 to collect the adherent cells adhering to the bubble 140 into the inner peripheral portion 110. In this manner, it is possible to selectively detach the adherent cells from the solid phase to collect the adherent cells by using the bubble 140 formed at the distal end portion 116. Furthermore, the collected cells may be released to another container or slide glass to move the adherent cells. Accordingly, the adherent cells can be subcultured, observed, or stained.

By supplying and aspirating gas from and into the flow channel of the inner peripheral portion 110, the channel member 100 can easily manipulate the fine object 35 in the liquid.

Although the adherent cell is exemplified as the fine object 35, the fine object 35 may be floating cells. When the fine object 35 is floating cells, without forming the bubble 140 in the distal end portion 116, the fluid (gas or liquid) contained in the flow channel of the inner peripheral portion 110 may be suctioned to bring the distal end portion 116 and the floating cells close to each other, and the fine object 35 may be taken into the flow channel of the inner peripheral portion 110. By this manipulation, observation and/or collection of the floating cells can be easily performed.

As an example of the manipulation of the adherent cells, the support unit 210 moves the channel member 100 along the surface of the solid phase while the bubble 140 are in contact with the adherent cells, but the manipulation of the adherent cells is not limited thereto. For example, the gas-liquid interface of the bubble 140 may be moved by increasing a volume of the bubble 140 while fixing the support unit 210, and the adherent cells may be attached to the bubble 140 and/or detached from the solid phase. Furthermore, by changing components of a medium containing the adherent cells or adjusting a time for forming a bubble, it is also possible to easily perform a manipulation of attaching the adherent cells to the bubble 140 and/or detaching the adherent cells from the solid phase, or pressing the adherent cells with the bubble 140.

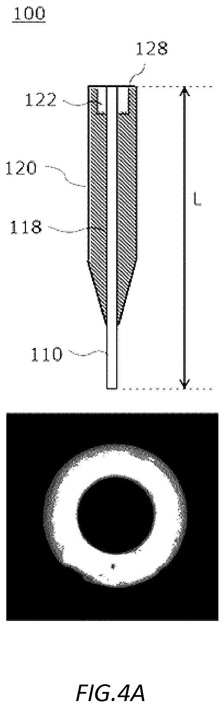

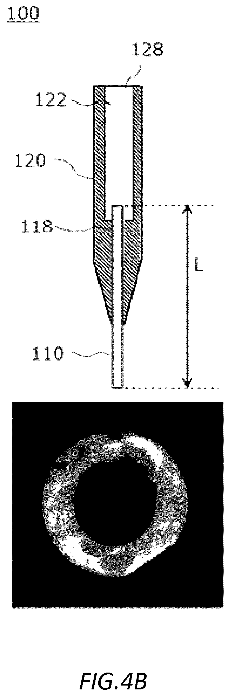

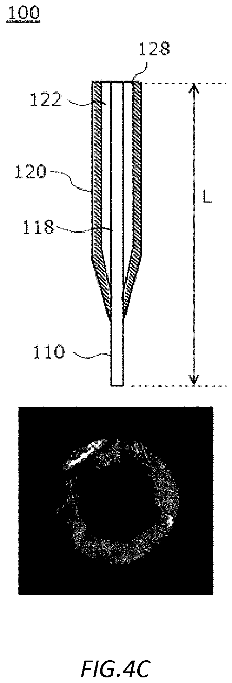

Figs. 4A, 4B, and 4C illustrate an example of the channel member 100 according to the present embodiment. In the channel members 100 of Figs. 4A, 4B, and 4C, borosilicate glass is used as the material forming the inner peripheral portion 110, and polycarbonate resin is used as the material forming the outer peripheral portion 120. The channel member 100 has the gap portion 122 between the inner peripheral portion 110 and the outer peripheral portion 120.

Figs. 4A and 4B each illustrate a bright-field image captured by locating the illumination unit 220 directly above each of two channel members 100 (vertically above the channel member 100 in the lengthwise direction) illustrated in Figs. 4A and 4B and illuminating the fine object 35 located directly below the distal end portion 116 through the channel member 100. The fine object 35 is more clearly observed when the fine object 35 located directly below the channel member 100 in Fig. 4B is imaged than when the fine object 35 located directly below the channel member 100 illustrated in Fig. 4A is imaged. Note that prior to imaging, the flow channel may be filled with liquid (for example, a buffer solution, a medium, or the like) in advance for the imaging.

By irradiating the fine object 35 with coherent light (here, the coherent light may be illumination light having an illumination NA of substantially zero and a coherence function having a sufficiently wide width compared to a wavelength) having a uniform phase from the illumination unit 220, a clear bright-field observation image can be obtained. When the channel member 100 is irradiated with light from the illumination unit 220, the light having passed through the inner peripheral portion 110 which is glass does not disturb the phase, and the fine object 35 directly below the inner peripheral portion 110 is irradiated with coherent light, so that a high-contrast observation image is obtained. On the other hand, the light having passed through the outer peripheral portion 120 which is resin is scattered and becomes incoherent light having a disturbed phase. When the light having passed through an inside of the outer peripheral portion 120 enters the inner peripheral portion 110, the phase of the light illuminating the fine object 35 is disturbed, an effective illumination NA increases (the width of the coherence function decreases), and a contrast decreases during bright-field observation, which causes an image of the fine object 35 to become unclear.

In this regard, by reducing an area of a portion where the inner peripheral portion 110 which is glass and the outer peripheral portion 120 which is resin are in contact with each other (which may be referred to as a contact area), the light having passed through the inside of the outer peripheral portion 120 is suppressed from entering the inner peripheral portion 110, and the fine object 35 is clearly observed.

The contact area may be 0.5 $mm^2$ or more and 4 $mm^2$ or less. The contact area may be 0.8 $mm^2$ or more and 3 $mm^2$ or less. The contact area may be 1 $mm^2$ or more and 2 $mm^2$ or less.

When the contact area is within the above range, the fine object 35 can be clearly observed. When the contact area between the inner peripheral portion 110 and the outer peripheral portion 120 exceeds 4 $mm^2$, an amount of the light having passed through the inside of the outer peripheral portion 120 and entering the inner peripheral portion 110 increases, and it is difficult to clearly observe the fine object 35. When the contact area between the inner peripheral portion 110 and the outer peripheral portion 120 is less than 0.5 $mm^2$, it becomes difficult to perform insert molding of the outer peripheral portion 120 for manufacturing the channel member 100.

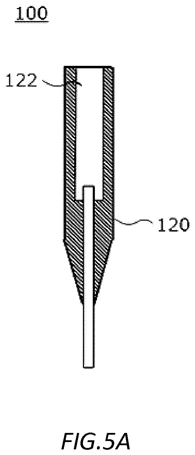

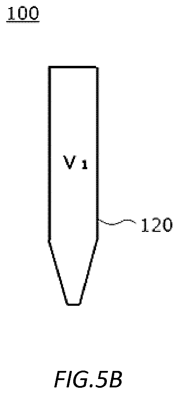

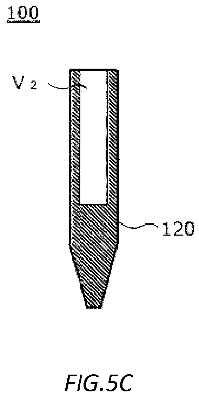

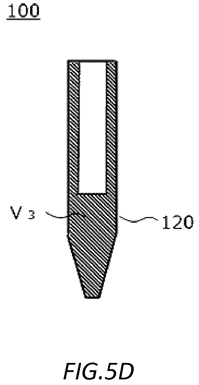

Figs. 5A, 5B, 5C, and 5D are diagrams for explaining a porosity and a filling rate of the outer peripheral portion 120 according to the present embodiment. The filling rate of the outer peripheral portion 120 may be 30% or more and 80% or less. The filling rate may be 40% or more and 70% or less. The filling rate may be 50% or more and 60% or less.

The porosity is defined as an index indicating a volume of the gap portion 122. The porosity is a proportion of a volume V2 of the gap portion 122 to an apparent volume V1 of the outer peripheral portion 120 including the gap portion 122.

In the channel member 100 illustrated in Fig. 5A, the apparent volume V1 of the outer peripheral portion 120 including the gap portion 122 means a volume of a space region surrounded by the material forming the outer peripheral portion 120 (a volume of a closed space illustrated in Fig. 5B). V1 also includes the volume of the gap portion 122. The volume V2 of the gap portion means a volume (a volume of a white portion illustrated in Fig. 5C) obtained by subtracting the volume of the space region occupied by the material forming the outer peripheral portion 120 (that is, an actual volume of the outer peripheral portion 120) from a volume of the region surrounded by the material forming the outer peripheral portion 120.

The porosity may be calculated by following Expression 1.

(Expression 1)

$$\text{Porosity (\%)} = 100 \times (V2/V1).$$

Instead of the porosity, the filling rate may be defined. The filling rate is a proportion of an actual volume V3 of the outer peripheral portion 120 not including the gap portion 122 (a volume of a shaded portion illustrated in Fig. 5D) to the apparent volume V1 of the outer peripheral portion 120 including the gap portion 122.

The filling rate may be calculated by following Expression 2.

(Expression 2)

$$\text{Filling rate (\%)} = 100 \times (V3/V1).$$

Since a sum of V2 and V3 is V1, the filling rate may be calculated by following Expression 3.

(Expression 3)

$$\text{Filling rate (\%)} = 100 - \text{porosity}.$$

When the filling rate is within a range of 30% or more and 80% or less, the fine object 35 can be clearly observed. When the filling rate exceeds 80%, the amount of the light having passed through the inside of the outer peripheral portion 120 and entering the inner peripheral portion 110 increases, and it is difficult to clearly observe the fine object 35. When the filling rate is less than 30%, the strength of the outer peripheral portion 120 cannot be maintained, and it is difficult to hold the inner peripheral portion 110 or to support the outer peripheral portion 120 by the support unit 210.

Here, a length L in the lengthwise direction of the inner peripheral portions 110 of the channel members 100 illustrated in Figs. 4A and 4C is 2.0 cm, and the inner peripheral portion 110 reaches the end portion 128 of the outer peripheral portion 120 on the side opposite to the distal end portion 116. The length L in the lengthwise direction of the inner peripheral portion 110 of the channel member 100 illustrated in Fig. 4B is 1.4 cm, which is shorter than the length in the lengthwise direction of the inner peripheral portions 110 of the channel members 100 illustrated in Figs. 4A and 4C, and does not reach the end portion 128.

The channel members 100 of Figs. 4A, 4B, and 4C are equal in the apparent volume V1 of the outer peripheral portion 120 including the gap portion 122, but differ in the volume V2 of the gap portion 122.

Here, as an index regarding the contact area, a contact area ratio is defined by following Expression 4.

Contact area ratio (%) =100×{(contact area between inner peripheral portion 110 and outer peripheral portion 120/cross-sectional area in plane orthogonal to lengthwise direction of inner peripheral portion 110). (Expression 4)

That is, the contact area ratio is a ratio of an area (contact area) of a contact portion between the inner peripheral portion 110 which is a glass tube and the outer peripheral portion 120 which is resin to a cross-sectional area of the inner peripheral portion 110 which is a glass tube. The contact area, the filling rate, and the contact area ratio of the channel member 100 used in Figs. 4A, 4B, and 4C are shown in Table 1.

(Table 1)

| FLOW CHANNEL MEMBER | CONTACT AREA ($mm^2$) | FILLING RATE (%) | CONTACT AREA RATIO (%) |
|---|---|---|---|
| FIG. 4A | 4.39 | 96.40 | 74628.8 |
| FIG. 4B | 2.51 | 74.90 | 42645.0 |
| FIG. 4C | 1.30 | 65.49 | 2.2164.7 |

Figs. 4A, 4B, and 4C each illustrate a bright-field image captured by locating the illumination unit 220 directly above each of three channel members 100 and imaging the fine object 35 located directly below the distal end portion 116 through the channel member 100.

In the imaging of the fine object 35 located directly below the channel member 100 in Fig. 4A, a contour of the fine object 35 is not recognized. However, in the imaging of the fine object 35 located directly below the channel member 100 in Fig. 4B, the contour is recognized. Furthermore, in the imaging of the fine object 35 located directly below the channel member 100 in Fig. 4C, the fine object 35 is more clearly observed. A reason for this is that, in the channel members 100 of Figs. 4B and 4C, the volume of the gap portion 122 is large (that is, a value of the filling rate is small) as compared with the channel member 100 of Fig. 4A, thereby suppressing an amount of light directly entering the inner peripheral portion 110 from the outer peripheral portion 120. Accordingly, it is considered that the disturbance of the phase of the light with which the illumination unit 220 illuminates the fine object 35 is suppressed.

The contact area ratio of the channel member 100 may be 50,000% (500 times) or less, or 30,000% (300 times) or less. When the contact area ratio is within a range of 50,000% (500 times) or less or 30,000% (300 times) or less, the fine object 35 can be clearly observed.

A partial region of the portion where the inner peripheral portion 110 and the outer peripheral portion 120 are in contact with each other may include a light shielding region where a light shielding member is arranged. In this case, in calculating the contact area ratio of the channel member 100, a region obtained by excluding the light shielding region from the contact area may be regarded as the contact area between the inner peripheral portion 110 and the outer peripheral portion 120.

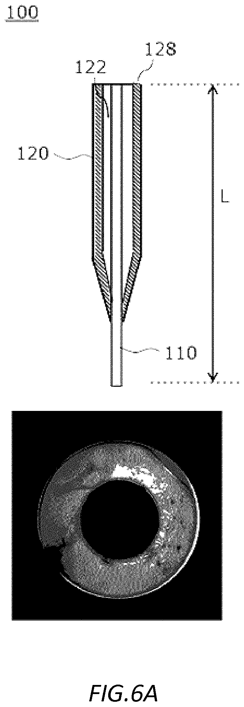

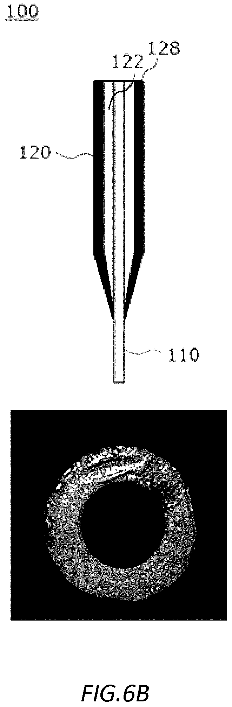

Figs. 6A and 6B illustrate another example of the channel member 100 according to the present embodiment. The light shielding member may be arranged on at least one of the inner peripheral portion 110 or the outer peripheral portion 120. As an example, the light shielding member is arranged in at least a portion of the inner peripheral portion 110 or the outer peripheral portion 120 where the inner peripheral portion 110 and the outer peripheral portion 120 are in contact with each other. Accordingly, the light having passed through the inside of the outer peripheral portion 120 is prevented from entering the inner peripheral portion 110. As an example, the light shielding member is arranged on an incident surface on which light is incident in the outer peripheral portion 120. As an example, the light shielding member is arranged on an incident surface on which the light is incident in the outer peripheral portion 120, in both end portions of the outer peripheral portion 120 in the lengthwise direction. Accordingly, the light entering the outer peripheral portion 120 is reduced, and the light having passed through the inside of the outer peripheral portion 120 is prevented from entering the inner peripheral portion 110.

For example, as the light shielding member, a light absorber and/or a light scatterer may be included in at least one of both end portions of the outer peripheral portion 120 in the lengthwise direction. Furthermore, for example, in place of/in addition to the light absorber and/or the light scatterer, a light shielding sheet may be provided as the light shielding member in at least a part between the inner peripheral portion 110 and the outer peripheral portion 120.

The light absorber absorbs light passing through the inside of the outer peripheral portion 120 to prevent the light from entering the inner peripheral portion 110 from the outer peripheral portion 120. A type of the light absorber is not limited as long as light can be absorbed. For example, as the light absorber, carbon black particles or metal oxide particles such as magnetite particles can be used. A color of the light absorber is not limited to black, and any dark color such as gray, dark blue, deep green, or dark brown may be used.

The light scatterer scatters light passing through the inside of the outer peripheral portion 120 in multiple directions to prevent the light from entering the inner peripheral portion 110 from the outer peripheral portion 120. Specifically, when the light having passed through the inside of the resin of the outer peripheral portion 120 is scattered in multiple directions by the light scatterer, an amount of the light entering the inner peripheral portion 110 decreases, thereby preventing the light from entering the inner peripheral portion 110 from the outer peripheral portion 120. A type of the light scatterer is not limited as long as light can be scattered.

As the light scatterer, inorganic particles or organic particles which effectively scatter light in a visible light region may be used. For example, white paint, titanium dioxide particles, zinc oxide particles, silicon dioxide particles, calcium carbonate particles, acrylic particles, or the like may be used as the light scatterer, but the light scatterer is not limited thereto.

The channel member 100 illustrated in Fig. 6A uses a same material as that of the channel member illustrated in Fig. 4A. The channel member 100 illustrated in Fig. 6B uses the same material as that of the channel member illustrated in Fig. 4A, but is different in that a polycarbonate resin mixed at a proportion of 0.018 weight percent with black paint (including carbon black particles) which is the light shielding member is used as the outer peripheral portion 120. In the channel member 100 illustrated in Fig. 6B, the black paint is contained in the outer peripheral portion 120, so that the outer peripheral portion 120 is blackened.

Figs. 6A and 6B each illustrate a bright-field image obtained by locating the illumination unit 220 directly above each of two channel members 100 (vertically above the channel member 100 in the lengthwise direction) illustrated in Figs. 6A and 6B and imaging the fine object 35 located directly below the distal end portion 116 through the channel member 100. The fine object 35 is more clearly observed when the fine object 35 located directly below the channel member 100 in Fig. 6B is imaged than when the fine object 35 located directly below the channel member 100 illustrated in Fig. 6A is imaged.

Light having passed through resin is scattered and a phase is disturbed. When the light having passed through the inside of the outer peripheral portion 120 which is resin enters the inner peripheral portion 110, the phase of the light illuminating the fine object 35 irradiated from the illumination unit 220 is disturbed, and the contrast decreases during the bright-field observation, which causes the image of the fine object 35 to become unclear. In this regard, when the resin is mixed with black paint containing carbon black particles, the light having passed through the inside of the outer peripheral portion 120 is absorbed by the carbon black particles, and the outer peripheral portion 120 is shielded from light. Accordingly, the light having passed through the inside of the outer peripheral portion 120 is suppressed from entering the inner peripheral portion 110, and the fine object 35 is clearly observed.

The light shielding sheet may be a sheet-like or film-like sheet coated with aluminum, a metal oxide, or the like, or may be one coated with black paint containing carbon black particles, but is not limited thereto as long as the light shielding sheet has a function of shielding light. Instead of the embodiment illustrated in Fig. 6B, for example, by applying black paint containing carbon black particles to at least a part of the resin of the outer peripheral portion 120 on a side facing the inner peripheral portion 110, the outer peripheral portion 120 can be shielded from light.

Instead of/in addition to shielding the outer peripheral portion 120 from light, the inner peripheral portion 110 may be shielded from light. For example, by applying black paint containing carbon black particles to at least a part of the inner peripheral portion 110 on the side facing the outer peripheral portion 120, the inner peripheral portion 110 can be shielded from light.

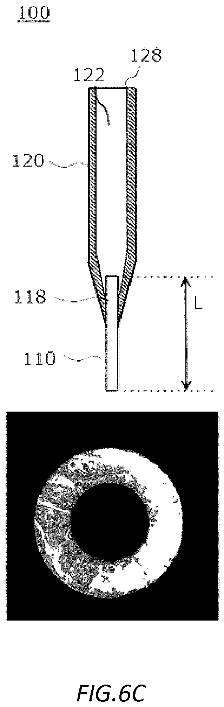

Fig. 6C illustrates another example of the channel member 100 according to the present embodiment. The length L of the inner peripheral portion 110 in the lengthwise direction may be longer, equal, or significantly shorter than the length of the outer peripheral portion 120 in the lengthwise direction (the longitudinal direction in the drawing). For example, the length of the inner peripheral portion 110 may be twice, equal to, 1/2 or less, 1/3 or less, 1/4 or less, or 1/5 or less of the length of the outer peripheral portion 120 in the lengthwise direction, but is not limited thereto.

As an example, the length L of the inner peripheral portion 110 of the channel member 100 in the lengthwise direction may be 1 mm or more and 50 mm or less. The length L of the inner peripheral portion 110 in the lengthwise direction may be 5 mm or more and 30 mm or less. The length L of the inner peripheral portion 110 in the lengthwise direction may be 10 mm or more and 20 mm or less.

When the length L of the inner peripheral portion 110 in the lengthwise direction is within the above range, the fine object 35 can be clearly observed. When the length L of the inner peripheral portion 110 in the lengthwise direction exceeds 50 mm, the light passing through the inside of the flow channel is reflected inside the flow channel, and it is difficult to clearly observe the fine object 35. When the length L of the inner peripheral portion 110 in the lengthwise direction is less than 1 mm, it is difficult to perform insert molding of the outer peripheral portion 120 for manufacturing the channel member 100.

Fig. 6C illustrates an example of the channel member 100. The channel member 100 in Fig. 6C and the channel member 100 in Fig. 6A use a same material, but differ from each other in the length L of the inner peripheral portion 110 in the lengthwise direction. The length L in the lengthwise direction of the inner peripheral portion 110 of the channel member 100 in Fig. 6A is 20 mm, and the inner peripheral portion 110 reaches the end portion 128 on the side opposite to the distal end portion 116 of the outer peripheral portion 120. The length L in the lengthwise direction of the inner peripheral portion 110 of the channel member 100 in Fig. 6C is 0.7 mm, which is shorter than the length in the lengthwise direction of the inner peripheral portion 110 of the channel member 100 in Fig. 6A and does not reach the end portion 128.

Fig. 6C illustrates a phase difference image obtained by locating the illumination unit 220 directly above the channel member 100 and imaging the fine object 35 located directly below the distal end portion 116 through the channel member 100. The fine object 35 is more clearly observed when the fine object 35 located directly below the channel member 100 in Fig. 6C is imaged than when the fine object 35 located directly below the channel member 100 in Fig. 6A is imaged.

When the length L of the inner peripheral portion 110 in the lengthwise direction is longer than 50 mm, it is considered that light is excessively reflected within the flow channel, and the fine object 35 is hardly observed clearly. Therefore, by shortening the length L of the inner peripheral portion 110 in the lengthwise direction to 50 mm or less, the light having passed through the inside of the outer peripheral portion 120 is suppressed from being reflected within the inner peripheral portion 110, and the fine object 35 is clearly observed. For example, the length of the inner peripheral portion 110 in the lengthwise direction may be 1/2 or less of the length of the outer peripheral portion 120 in the lengthwise direction.

A volume occupied by the inner peripheral portion 110 is represented by a product of a cross-sectional area of the flow channel constituting the inner peripheral portion 110 (the area of the cross section in the plane orthogonal to the lengthwise direction of the inner peripheral portion 110) and the length L of the inner peripheral portion 110. When the length L of the inner peripheral portion 110 in the lengthwise direction decreases, the volume occupied by the inner peripheral portion 110 decreases, and thus it is possible to reduce the amount of liquid suctioned into the flow channel of the inner peripheral portion 110 when the fine object 35 is manipulated.

As the amount of liquid suctioned into the flow channel of the inner peripheral portion 110 decreases, the amount of liquid introduced in the next manipulation (for example, molecular biological analysis of a collected organism) can be reduced, which is advantageous when the manipulation is performed.

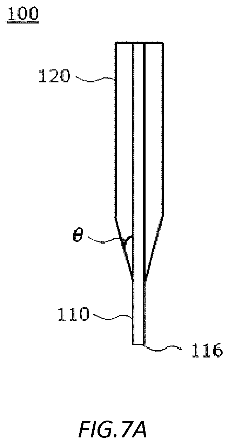

Fig. 7A is a diagram for explaining a taper angle of the outer peripheral portion 120 according to the present embodiment. A taper angle θ means an angle formed by the lengthwise direction of the inner peripheral portion 110 and a side surface of a tapered portion of the outer peripheral portion 120 in which the area of the cross section in the plane orthogonal to the lengthwise direction of the inner peripheral portion 110 decreases toward the distal end portion 116.

The taper angle θ may be 4 degrees or more and 80 degrees or less. The taper angle θ may be 6 degrees or more and 50 degrees or less. The taper angle θ may be 8 degrees or more and 20 degrees or less. The taper angle θ may be 8.5 degrees or more and 10 degrees or less. When the taper angle θ is within the above range, the fine object 35 can be clearly observed.

When the taper angle is 4 degrees or less, the amount of the light having passed through the inside of the outer peripheral portion 120 and entering the inner peripheral portion 110 increases, and it is difficult to clearly observe the fine object 35. Furthermore, when a diameter of the channel member 100 is large, the length of the channel member 100 in the lengthwise direction becomes considerably large, and it becomes difficult to manipulate the channel member 100. When the taper angle exceeds 80 degrees, the illumination unit 220 (for example, an auxiliary illumination described later) obstructs irradiation of the vicinity directly below the inner peripheral portion 110, and it is difficult to clearly observe the fine object 35 existing in the vicinity directly below the inner peripheral portion 110. Furthermore, since the taper angle is close to 90 degrees, incoherent light generated by the light passing through the outer peripheral portion 120 directly illuminates the fine object 35 existing in the vicinity directly below the inner peripheral portion 110, and it is difficult to clearly observe the fine object 35.

The cross section of the distal end portion 116 of the channel member 100 may be smooth without scratches or irregularities. Specifically, a surface roughness of the cross section of the distal end portion 116 perpendicular to the lengthwise direction of the inner peripheral portion 110 (alternatively, which may be a cross section inclined from the plane perpendicular to the lengthwise direction) may have an arithmetic average roughness (Ra) of 20 μm or less and/or a maximum height (Rz) of 30 μm or less.

The arithmetic average roughness may be considered as an index of an average value of height differences in the cross section. The maximum height may be considered as an index of a maximum value of the height difference in the cross section. When the arithmetic average roughness exceeds 20 μm or the maximum height exceeds 30 μm, the cross section of the distal end portion 116 is not smooth, so that there is possibility that the bubble 140 cannot be appropriately formed, and the fine object 35 cannot be appropriately manipulated. When the arithmetic average roughness exceeds 20 μm or the maximum height exceeds 30 μm, an influence of refraction of light in the cross section of the distal end portion 116 increases, and there is possibility that the illumination unit 220 cannot uniformly illuminate the fine object 35.

When the cross section of the distal end portion 116 of the channel member 100 has irregularities, an average value of distances between the irregularities may be 5 times or more, or 10 times or more the wavelength. When the distance between the irregularities is 5 times or less the wavelength, light undergoes diffuse reflection, and the contrast decreases during the bright-field observation of the fine object 35, which causes the image of the fine object 35 to become unclear.

Measurement conditions and calculation methods of the arithmetic average roughness and/or the maximum height may be in accordance with "ISO 25178 surface texture (surface roughness measurement)". The arithmetic average roughness may be measured in a non-contact manner by using a 3D measurement laser microscope or the like.

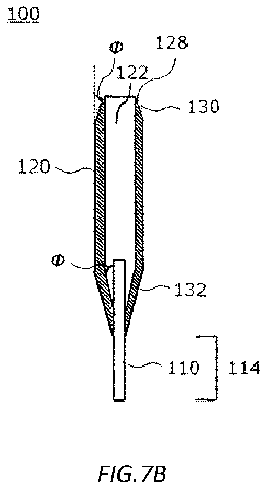

Fig. 7B illustrates another example of the channel member 100 according to the present embodiment. In the channel member 100 illustrated in Fig. 7B, inclinations are provided in an outer edge portion 130 of the outer peripheral portion 120 at the end portion 128 and an outer peripheral bottom portion 132 of the gap portion 122 of the outer peripheral portion 120 on the distal end region 114 side. Light illuminating from directly above the channel member 100 is easily incident on a planar portion of the end portion 128.

In this regard, by providing an inclination in at least one of the outer edge portion 130 or the outer peripheral bottom portion 132, light hardly enters the outer peripheral portion 120, and it is possible to reduce light entering the inner peripheral portion 110 from the outer peripheral portion 120. Accordingly, the fine object 35 can be clearly observed. An inclination angle φ, which is an angle formed by the provided inclined surface and the lengthwise direction of the channel member 100, may be within a range of a same angle as the taper angle θ, for example, 4 degrees or more and 80 degrees or less, 6 degrees or more and 50 degrees or less, 8 degrees or more and 20 degrees or less, or 8.5 degrees or more and 10 degrees or less, and the inclination angle θ, which is an angle formed by the lengthwise direction of the channel member 100, may be a negative angle (for example, -80 degrees or more and -4 degrees or less, -50 degrees or more and -6 degrees or less, -20 degrees or more and -8 degrees or less, -10 degrees or more and -8.5 degrees or less, or the like).

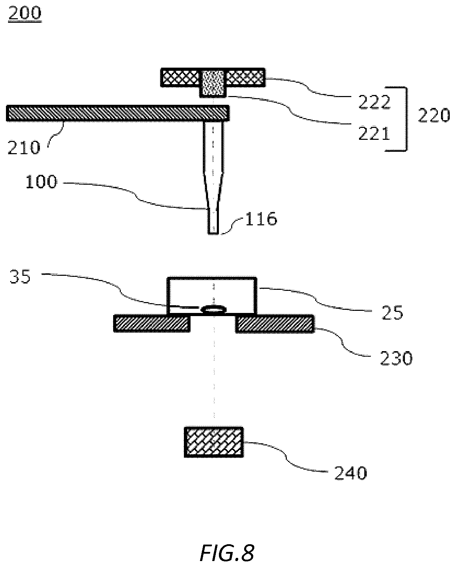

Fig. 8 illustrates an example of a configuration of the illumination unit 220 of the fine object manipulation device 200 according to the present embodiment (excluding the control unit 250). In Fig. 8, the illumination unit 220 may have at least two types of illuminations, that is, a main illumination 221 and an auxiliary illumination 222.

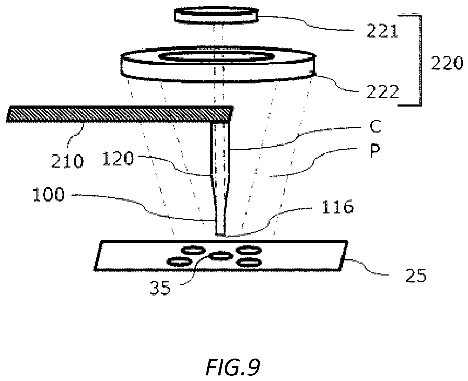

Fig. 9 is a perspective view of the fine object manipulation device 200 of Fig. 8 (excluding the imaging unit 240).

The main illumination 221 illuminates the channel member 100 from directly above (C of Fig. 9 illustrates an optical path of the main illumination). The main illumination 221 may irradiate the channel member 100 with light along the lengthwise direction of the inner peripheral portion 110 (not illustrated). The light incident on the inner peripheral portion 110 from the main illumination 221 may be emitted from the distal end portion 116. The main illumination 221 may radiate coherent light. The coherent light may be light from a halogen lamp, light from a mercury lamp, light from an LED, or laser light, but is not limited thereto.

A shape of the main illumination 221 is not particularly limited, but may be a disc shape, a ring shape, or a rectangular shape. When the main illumination 221 radiates coherent light, the light is incident on the inner peripheral portion 110, and a bright-field image of the fine object 35 located directly below the inner peripheral portion 110 can be clearly observed.

The auxiliary illumination 222 illuminates the fine object 35 from an oblique direction of the channel member 100 (P in Fig. 9 indicates an optical path of the auxiliary illumination). The auxiliary illumination 222 may be a ring illumination that radiates ring-shaped light that circumferentially surrounds the channel member 100. Since the auxiliary illumination 222 is the ring illumination, bright-field images of the fine object 35 located in the vicinity directly below and on an inner side directly below the inner peripheral portion 110 can be evenly and clearly observed.

The auxiliary illumination 222 is an oblique ring illumination. Fig. 9 illustrates an example in which the auxiliary illumination 222 is the oblique ring illumination. Since the auxiliary illumination 222 is the oblique ring illumination, the fine object 35 can be observed with higher contrast. In addition, a similar effect can be obtained in a ring illumination without oblique light, but the oblique ring illumination can illuminate the fine object 35 more efficiently.

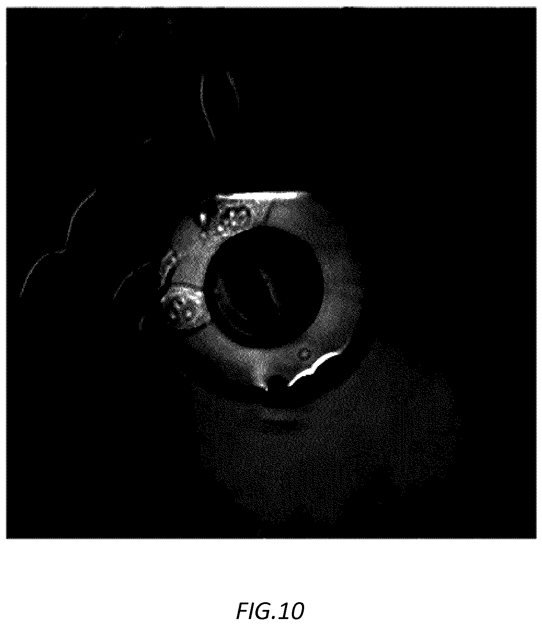

Fig. 10 illustrates an example of the bright-field image of the fine object according to the present embodiment. By adjusting light amounts of the main illumination 221 and the auxiliary illumination 222 such that brightness directly below the inner peripheral portion 110 and in the vicinity directly below and in the inner side directly below the inner peripheral portion 110 become uniform, it is possible to clearly observe all the fine objects 35 within a field of view as illustrated in Fig. 10. The light amounts may be adjusted automatically by an information processing apparatus (for example, the control unit 250) such as a computer connected to the illumination unit 220, or may be adjusted manually by the observer.

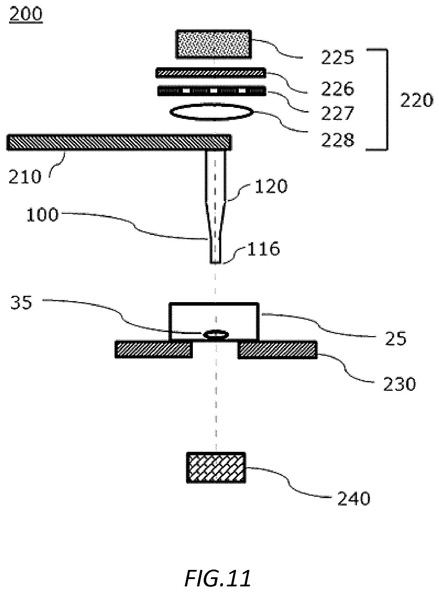

Fig. 11 illustrates another example of the configuration of the illumination unit 220 of the fine object manipulation device 200 according to the present embodiment (excluding the control unit 250). In Fig. 11, the illumination unit 220 includes a light source 225. The illumination unit 220 may include a diaphragm 226, a filter 227, and a condenser lens 228 in order to divide the light source 225 into two of light for illuminating from directly above the fine object 35 (sometimes referred to as center light) and light for illuminating the vicinity directly below the fine object 35, that is, a region surrounding a vicinity of the center light (sometimes referred to as peripheral light).

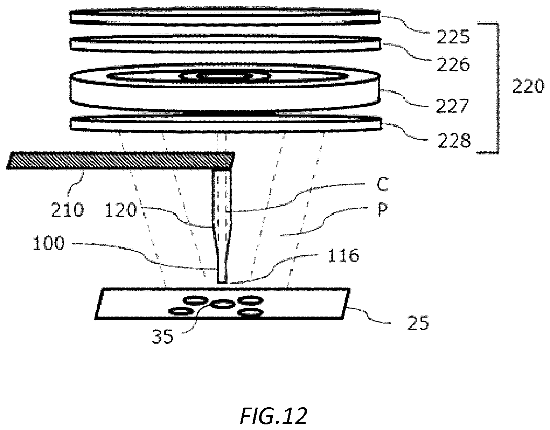

Fig. 12 illustrates a perspective view of the fine object manipulation device 200 of Fig. 11 (excluding the imaging unit 240).

The light source 225 may illuminate the fine object 35 from directly above the channel member 100, and may illuminate a region directly below the inner peripheral portion 110, and the vicinity directly below and the inner side directly below the inner peripheral portion 110. The light source 225 may radiate coherent light.

The diaphragm 226 adjusts the amount of light from the light source 225. By narrowing the diaphragm 226, the light from the light source 225 can be limited to only the center light (C of Fig. 12 indicates an optical path of the center light). By opening the diaphragm 226, the light from the light source 225 can include the center light and the peripheral light (P in Fig. 12 indicates an optical path of the peripheral light). In particular, a light amount of the peripheral light can be adjusted by adjusting an aperture of the diaphragm 226. The diaphragm 226 may be adjusted automatically by an information processing apparatus (for example, the control unit 250) such as a computer connected to the illumination unit 220, or may be adjusted manually by the observer.

The filter 227 divides the light from the light source 225 into the center light and the peripheral light. The filter 227 may have a circular hole in a central portion and an annular hole in a peripheral portion present in a vicinity of the central portion. The light of the light source 225 becomes the center light when passing through the central portion of the filter 227, and becomes the peripheral light when passing through the peripheral portion of the filter 227. The hole in the central portion of the filter 227 may have a diameter of 10 mm or less such that the center light does not illuminate other than the channel member 100.

The condenser lens 228 may condense the center light from the filter 227 and cause the center light to be incident on the inner peripheral portion 110 (not illustrated). The center light incident on the inner peripheral portion 110 is emitted from the distal end portion 116 and illuminates the fine object 35 from directly above (C of Fig. 12 illustrates the optical path of the center light). The condenser lens 228 may condense the peripheral light from the filter 227 and illuminate the fine object 35 from the oblique direction (P in Fig. 12 indicates the optical path of the peripheral light). When the light source 225 radiates coherent light, the light illuminating the fine object 35 from directly above may include the coherent light, and the light illuminating the fine object 35 from the oblique direction may include incoherent light.

The illuminance for illuminating the fine object 35 can be adjusted in a manner that brightness of the center light is adjusted by adjusting brightness of the light source 225 itself by the illuminance adjustment unit 260, and brightness of the peripheral light is adjusted by the diaphragm 226. In this manner, even when the illumination unit 220 is one light source 225, the illumination unit 220 further includes the diaphragm 226, the filter 227, and the condenser lens 228, so that the light source 225 that illuminates the fine object 35 can be divided into two types of illuminations of the center light and the peripheral light. It is possible to reduce a cost for the light source 225 to radiate coherent light. Since the illumination unit 220 further includes the diaphragm 226, the filter 227, and the condenser lens 228, it is possible to adjust an amount of coherent light in the center light and an amount of incoherent light in the peripheral light.

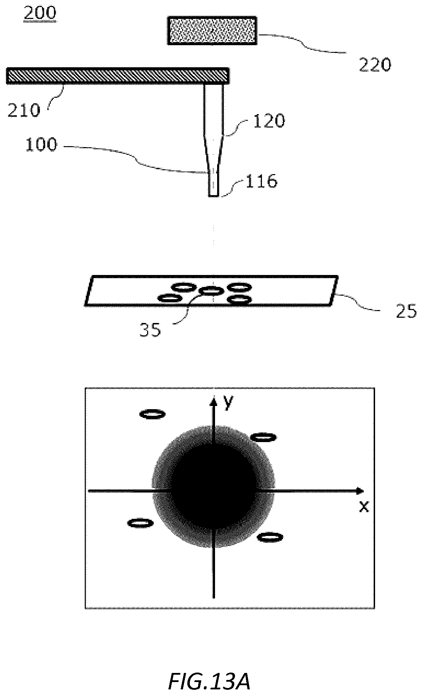

Fig. 13A is an example of the fine object manipulation device 200 according to the present embodiment and the image of the fine object 35 captured by the fine object manipulation device 200. In the image of Fig. 13A, an x axis and a y axis indicate orthogonal axes of a two-dimensional coordinate system with a position directly below the channel member 100 as an origin.

As illustrated in Fig. 13A, the imaging unit 240 (not illustrated) may generate an image in which the fine object 35 directly below the channel member 100 is imaged darker than the surrounding fine object 35. This is considered to be partly because a part of the light from the illumination unit 220 is blocked by the outer peripheral portion 120 of the channel member 100, and the region directly below the channel member 100 becomes a shadow of the outer peripheral portion 120.

In such a case, in order to generate an image in which the fine object 35 directly below the channel member 100 is as bright as the surrounding fine object 35, the sensitivity of the imaging element may be adjusted by controlling the sensitivity adjustment unit 270. The sensitivity adjustment unit 270 adjusts the sensitivity of the imaging element located directly below the channel member 100 among the imaging elements of the imaging unit 240, based on the sensitivity of the imaging element located in the vicinity directly below the channel member 100. The sensitivity adjustment unit 270 may adjust the sensitivity of the imaging element located directly below the channel member 100 among the imaging elements of the imaging unit 240 to be higher than the sensitivity of the imaging element located in the vicinity directly below the channel member 100.

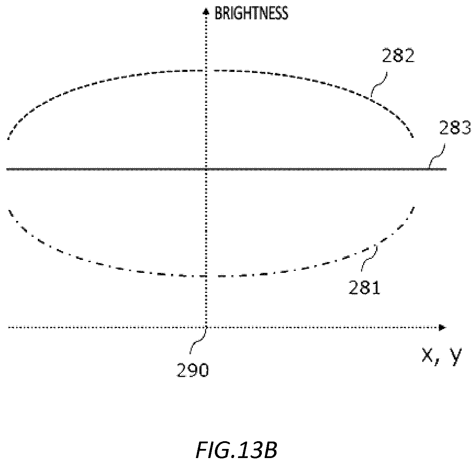

Fig. 13B is a diagram for explaining the above adjustment. A point 290 indicates a position directly below the channel member 100, a horizontal axis (x, y) indicates a distance from the point 290, and a vertical axis indicates relative brightness. A curve 281 indicates relative brightness of the image captured by the imaging unit 240. The curve 281 indicates that the brightness is the darkest directly below the channel member 100 (point 290).

Specifically, the sensitivity adjustment unit 270 may adjust the sensitivity automatically or manually by the observer such that the sensitivity of the imaging element located directly below the channel member 100 (point 290) is maximized, and the sensitivity of the imaging element located in the vicinity directly below the channel member 100 is gradually increased toward the region directly therebelow. A curve 282 indicates a relative value of the sensitivity of the imaging element when the sensitivity is adjusted in such a manner. A straight line 283 indicates the brightness of the image obtained by the adjustment. With this adjustment, the region directly below the channel member 100 can be brightened to a same extent as the vicinity directly therebelow.

**In** such a case, the luminance adjustment unit 280 may be controlled by another control method to adjust the luminance such that the image of the fine object 35 directly below the channel member 100 is as bright as the surrounding fine object 35. The luminance adjustment unit 280 adjusts the luminance of the image located directly below the channel member 100 in the image captured by the imaging unit 240, based on the luminance of the image located in the vicinity directly below the channel member 100. The luminance adjustment unit 280 may adjust the luminance of the image located directly below the channel member 100 in the image captured by the imaging unit 240 to be amplified to be higher than the luminance of the image located in the vicinity directly below the channel member 100.

Specifically, in Fig. 13B, the luminance adjustment unit 280 may adjust the luminance automatically or manually by the observer such that the luminance of the image located directly below the channel member 100 (point 290) is amplified to a highest level, and the luminance of the image located in the vicinity directly below the channel member 100 is amplified to gradually increase toward the region directly therebelow. The curve 282 indicates a relative value of an amplification ratio of the luminance when adjusted in that manner. The straight line 283 indicates the brightness of the image obtained by the adjustment. Even with this adjustment, the region directly below the channel member 100 can be brightened to the same extent as the vicinity directly therebelow.

Such adjustment of the sensitivity adjustment unit 270 and/or the luminance adjustment unit 280 may be performed following the operation of the channel member 100. By adjusting the sensitivity or luminance following the operation of the channel member 100, it is possible to observe an image, in which the region directly below the channel member 100 is bright, in real time.

By controlling the sensitivity adjustment unit 270 and/or the luminance adjustment unit 280 in this manner, the image of the fine object 35 directly below the channel member 100 becomes as bright as the image of the surrounding fine object 35, and an image can be obtained with uniform brightness for both the fine object 35 located directly below the channel member 100 and the fine object 35 located in the vicinity directly therebelow.

While the present invention has been described by way of the embodiments, the technical scope of the present invention is not limited to the above-described embodiments. It is apparent to persons skilled in the art that various alterations or improvements can be made to the above-described embodiments. It is also apparent from the scope of the claims that the embodiments added with such alterations or improvements can be included in the technical scope of the present invention.

The operations, procedures, steps, and stages of each process performed by an apparatus, system, program, and method shown in the claims, embodiments, or diagrams can be performed in any order as long as the order is not indicated by "prior to," "before," or the like and as long as the output from a previous process is not used in a later process. Even if the process flow is described by using phrases such as "first", "then" or the like in the scope of the claims, embodiments, or diagrams, it does not necessarily mean that the process must be performed in this order.

EXPLANATION OF REFERENCES

25: container;
35: fine object;
100: channel member;
110: inner peripheral portion;
112: inner region;
114: distal end region;
116: distal end portion;
118: protruding portion;
120: outer peripheral portion;
122: gap portion;
124: surrounding portion;
128: end portion;
130: outer edge portion;
132: outer peripheral bottom portion;
140: bubble;
145: liquid;
200: fine object manipulation device;
210: support unit;
220: illumination unit;
221: main illumination;
222: auxiliary illumination;
225: light source;
226: diaphragm;
227: filter;
228: condenser lens;
230: placement unit;
240: imaging unit;
250: control unit;
255: flow channel manipulation unit;
260: illuminance adjustment unit;
270: sensitivity adjustment unit;
280: luminance adjustment unit;
281: curve;
282: curve;
283: straight line; and
290: point.

**Claims**

**1.** A channel member which is a member including a flow channel which allows fluid to pass through to enable manipulation of a fine object, the channel member comprising:

an inner peripheral portion which is in contact with the fluid; and
an outer peripheral portion which holds the inner peripheral portion and has a lower melting temperature than that of the inner peripheral portion.

**2.** The channel member according to claim 1, wherein

a material forming the outer peripheral portion has a lower Young's modulus at 25°C than that of a material forming the inner peripheral portion.

**3.** The channel member according to claim 1, wherein
a material forming the inner peripheral portion has a larger light transmittance value at all wavelengths of 400 nm to 700 nm than that of a material forming the outer peripheral portion.

**4.** The channel member according to claim 1, wherein

a material forming the inner peripheral portion is glass, and
a material forming the outer peripheral portion is resin.

**5.** The channel member according to claim 1, wherein
the fine object is a phase object.

**6.** The channel member according to claim 1, wherein
at least one of the outer peripheral portion or the inner peripheral portion includes a light shielding member.

**7.** The channel member according to claim 6, wherein
the light shielding member is arranged on an incident surface on which light is incident in the outer peripheral portion.

**8.** The channel member according to claim 6, wherein
the light shielding member is arranged at a portion where the outer peripheral portion and the inner peripheral portion are in contact with each other.

**9.** The channel member according to claim 6, wherein
the light shielding member is black paint.

**10.** The channel member according to claim 1, wherein
a ratio of an area of a portion where the outer peripheral portion and the inner peripheral portion are in contact with each other to a cross-sectional area in a plane orthogonal to a lengthwise direction of the inner peripheral portion is 500 times or less.

**11.** The channel member according to claim 1, wherein

the inner peripheral portion has an elongated tubular shape that allows internal flow of the fluid, and includes a distal end region having a certain length including a distal end portion close to the fine object, and an inner region other than the distal end region, and
the outer peripheral portion surrounds the inner peripheral portion in the inner region, and a cross-sectional area of a cross section in a plane orthogonal to a lengthwise direction of the inner peripheral portion decreases toward the distal end portion.

**12.** The channel member according to claim 11, wherein
the outer peripheral portion has a portion surrounding the inner peripheral portion without being in contact with the inner peripheral portion, whereby a gap portion serving as a gap exists between the outer peripheral portion and the inner peripheral portion.

**13.** The channel member according to claim 12, wherein
a proportion of an actual volume of the outer peripheral portion not including the gap portion to an apparent volume of the outer peripheral portion including the gap portion is 30 to 80%.

**14.** A fine object manipulation device comprising:

the channel member according to any one of claims 1 to 13;
a support unit which supports the outer peripheral portion of the channel member; and
an illumination unit which irradiates the fine object with light at least partially through the channel member and is arranged above the support unit.

**15.** The fine object manipulation device according to claim 14, wherein

the illumination unit includes a main illumination which irradiates the channel member with light along a lengthwise direction of the inner peripheral portion, and
light incident on the inner peripheral portion from the main illumination is emitted from a distal end portion of the inner peripheral portion to illuminate the fine object from directly above.

**16.** The fine object manipulation device according to claim 15, wherein

the illumination unit further includes
an auxiliary illumination which radiates ring-shaped light circumferentially surrounding the channel member and illuminates the fine object from an oblique direction.

**17.** The fine object manipulation device according to claim 14, wherein

the illumination unit includes
a light source,
a filter which divides light from the light source into center light and peripheral light surrounding a vicinity of the center light, and
a condenser lens which condenses the center light from the filter to cause the center light to be incident on the inner peripheral portion and emits light from a distal end portion of the inner peripheral portion to illuminate the fine object from directly above, and condenses the peripheral light to illuminate the fine object from an oblique direction.

**18.** The fine object manipulation device according to claim 14, wherein
the illumination unit radiates coherent light.

**19.** The fine object manipulation device according to claim 14, further comprising:

a placement unit on which the fine object is placed; and
an imaging unit which is arranged on a surface opposite to the channel member with respect to the placement unit and captures a magnified image of the fine object.

**20.** The fine object manipulation device according to claim 19, wherein
the imaging unit images a manipulation performed by the fluid on the fine object close to a distal end portion of the inner peripheral portion.

**21.** The fine object manipulation device according to claim 20, wherein
the manipulation is performed by a bubble formed and maintained at the distal end portion of the inner peripheral portion.

**22.** The fine object manipulation device according to claim 19, further comprising
an illuminance adjustment unit which adjusts illuminance of the illumination unit.

**23.** The fine object manipulation device according to claim 19, further comprising
an adjustment unit which adjusts sensitivity of an imaging element or luminance of an image located directly below the channel member among imaging elements of the imaging unit, based on sensitivity of an imaging element or luminance of the image located in a vicinity directly below the channel member.

200
220
210
100
116
35
25
230
FLOW CHANNEL MANIPULATION UNIT 255
ILLUMINANCE ADJUSTMENT UNIT 260
SENSITIVITY ADJUSTMENT UNIT 270
LUMINANCE ADJUSTMENT UNIT 280
240
250

*FIG.1*

100
122
128
120
112
118
124
110
114
116

FIG.2

110
120
100
116
210
128
145
140
25
35

FIG.3A

210
128
110
120
100
116
140
145
25
35

FIG.3B

110
120
210
128
100
116
145
25
35

*FIG.3C*

100
128
122
120
118
110
L

*FIG.4A*

100
128
122
120
118
L
110

*FIG.4B*

100
128
122
120
L
118
110

*FIG.4C*

100

122
120

*FIG.5A*

100
V 1
120

*FIG.5B*

100
V 2
120

FIG.5C

100
V 3
120

FIG.5D

100
128
122
120
L
110

*FIG.6A*

100
128
122
120
110

*FIG.6B*

100
128
122
120
118
L
110

FIG.6C

100
120
θ
110
116

FIG.7A

100
Φ
128
122
130
120
Φ
132
110
114

FIG.7B

200
222
220
221
210
100
116
35
25
230
240

FIG.8

221
220
222
C
P
210
120
100
116
35
25

*FIG.9*

*FIG.10*

200
225
226
227
228
220
210
120
100
116
35
25
230
240

*FIG.11*

225
226
220
227
228
C
210
120
P
100
116
25
35

*FIG.12*

200
220
210
120
100
116
25
35
y
x

*FIG.13A*

BRIGHTNESS
282
283
281
290
X, y

*FIG.13B*

## INTERNATIONAL SEARCH REPORT

International application No.
**PCT/JP2023/037882**

**A. CLASSIFICATION OF SUBJECT MATTER**

***C12M 1/26***(2006.01)i; ***B05C 5/00***(2006.01)i; ***B05C 11/00***(2006.01)i; ***B05C 11/10***(2006.01)i; ***C12M 1/28***(2006.01)i
FI: C12M1/26; C12M1/28; B05C11/00; B05C11/10; B05C5/00 101

According to International Patent Classification (IPC) or to both national classification and IPC

**B. FIELDS SEARCHED**

Minimum documentation searched (classification system followed by classification symbols)

C12M1/26; B05C5/00; B05C11/00; B05C11/10; C12M1/28

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | JP 3-46559 A (HITACHI CABLE LTD) 27 February 1991 (1991-02-27) claims, p. 2, lower right column, 2nd paragraph to p. 3, upper left column, 1st paragraph, fig. 1-3 | 1-10 |
| Y | | 1-23 |
| X | JP 2004-85292 A (HITACHI CHEM CO LTD) 18 March 2004 (2004-03-18) paragraphs [0001], [0014] | 1-10 |
| Y | | 1-23 |
| X | JP 57-141554 A (FURUKAWA ELECTRIC CO LTD) 01 September 1982 (1982-09-01) claims, p. 1, left column 3rd paragraph to p. 1, right column, 2nd paragraph | 1-10 |
| Y | | 1-23 |
| Y | WO 2022/065460 A1 (NIKON CORPORATION) 31 March 2022 (2022-03-31) abstract, claims, paragraphs [0001], [0027]-[0065], [0073]-[0078], [0286]-[0290], fig. 1A, 1B, 2A, 2B, 4, 13I, 13J | 1-23 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **25 December 2023** | **09 January 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2023/037882**

| Patent document cited in search report | Publication date (day/month/year) | Patent family member(s) | Publication date (day/month/year) |
|---|---|---|---|
| JP 3-46559 A | 27 February 1991 | (Family: none) | |
| JP 2004-85292 A | 18 March 2004 | US 2007/0205105 A1 paragraphs [0002], [0049] | |
| JP 57-141554 A | 01 September 1982 | (Family: none) | |
| WO 2022/065460 A1 | 31 March 2022 | EP 4219676 A1 abstract, claims, paragraphs [0001], [0028]-[0066], [0074]-[0079], [0340]-[0344], fig. 1A, 1B, 2A, 2B, 4, 13I, 13J | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2016000007 A